# EUROPEAN PATENT SPECIFICATION

(11) **EP 3 120 885 B1**
(45) Date of publication and mention of the grant of the patent: **31.07.2024**
(21) Application number: 15765208.2
(22) Date of filing: 12.03.2015
(51) Int. Cl.: G16H 40/63, G16H 20/17, A61M 5/145, A61M 5/142

(54) **LIQUID DELIVERY PUMP**
FLÜSSIGKEITSAUSGABEPUMPE
POMPE DE DISTRIBUTION DE LIQUIDE

(30) Priority: 20.03.2014 JP 2014058596
(43) Date of publication of application: 25.01.2017
(73) Proprietor: Terumo Kabushiki Kaisha, Tokyo 151-0072 (JP)
(72) Inventor: OSAWA, Yuko, Tokyo 163-1450 (JP); HASEGAWA, Eiji, Ashigarakami-gun Kanagawa 259-0151 (JP)
(74) Representative: KIPA AB
(86) International application number: PCT/JP2015/057383
(87) International publication number: WO 2015/141563

(56) References cited:
- DE-A1- 102005 028 080
- JP-A- 2005 529 638
- JP-A- 2008 520 373
- JP-A- 2008 546 435
- JP-A- 2010 514 502
- JP-A- 2013 542 414
- US-A1- 2010 094 202

## Description

### Technical Field

The present invention relates to a liquid delivery pump used in a medical field to deliver a drug into the body of a patient.

### Background Art

In a case where a drug such as intravenous anesthetic is delivered into the body of a patient on a medical site such as an intensive care unit, it is necessary to deliver the drug for a long time while appropriately adjusting a delivery flow rate according to an applied technique and conditions of the patient. An exemplary apparatus for delivering the drug correctly for a long time with a set delivery flow rate includes an infusion pump and a syringe pump. The setting of the delivery flow rate is performed by medical professionals who use infusion pumps and syringe pumps. Typically, the delivery flow rate at the time of delivery of a drug into the body of a patient has its upper limit value determined according to the type of drug. Specifically, there are different upper limit values for the delivery flow rates, namely, upper limit values allowable at introduction of a drug into the living body before efficacy of the drug is expressed, and upper limit values allowable for maintaining the efficacy of the drug after the efficacy of the drug is expressed at a specific site of the living body. Therefore, the delivery flow rate needs to be appropriately adjusted so as not to exceed the upper limit values determined for individual types of drugs, according to whether the efficacy of the drug is expressed, when the drug is delivered. Typically, adjustment of the delivery flow rate has been manually performed by medical professionals. This can be problematic in that adjustment of the delivery flow rate according to whether the efficacy of the drug is expressed might not always be performed at an appropriate timing.

To cope with such a problem, there has been developed, in recent years, a pump (hereinafter, referred to as a TCI pump) equipped with a function referred to as target controlled infusion (TCI) that automatically controls the delivery flow rate according to a drug concentration of the delivered drug in blood (for example, refer to Patent Literature 1). When drug delivery is performed using the TCI pump, a drug concentration of the drug in blood is set as a target, instead of the delivery flow rate. The TCI pump automatically adjusts the delivery flow rate such that the drug concentration of the drug in blood of the delivered drug reaches a target concentration and is maintained at the level. The drug concentration of the drug in blood is calculated from the delivery flow rate of the drug on the basis of simulation. By using the TCI pump, it is possible to deliver a drug for a long time while maintaining the drug concentration of the drug in blood at a target concentration without manually adjusting the delivery flow rate. DE 10 2005 028080 A1 and and US 2010/094202 A1 both disclose a device for the time controlled intravenous administering of the anesthetic propofol by means of a method used for determining an adequate dosage profile and adequately controlling an infusion pump as a metering apparatus. However, both documents do not disclose an interplay of two upper limits that are adjusted and iterated according to certain rules (target concentrations) as specified by the present invention.

### Citation List

### Patent Literature

Patent Literature 1: JP 2008-546435 A

### Summary of Invention

### Technical Problem

Unfortunately, the use of the TCI pump might cause delivery of a liquid with a delivery flow rate exceeding an upper limit value of the delivery flow rate, prescribed for individual types of drugs. For example, in a case where the target drug concentration in blood has been set to a level higher than an allowable drug concentration in blood at the start of delivery of the drug, there is a possibility that the drug is delivered at a delivery flow rate exceeding the upper limit value in order to promptly allow the drug concentration in blood to reach the target drug concentration in blood. Some TCI pumps can set an upper limit value for the delivery flow rate to prevent delivery of the drug at the delivery flow rate exceeding the upper limit value. These TCI pumps, however, have no function to perform liquid delivery while switching the upper limit value of the delivery flow rate according to whether efficacy of the drug is expressed, and thus, liquid delivery is not necessarily performed with an appropriate delivery flow rate. There is another problem that the upper limit value of the delivery flow rate might be set erroneously.

The present invention is made to overcome this problem, and an object of the present invention to provide a liquid delivery pump capable of, at delivery of a drug, delivering the drug with an appropriate delivery flow rate that does not exceed a predetermined upper limit value prescribed for individual types of drugs.

### Solution to Problem

The invention is defined by the accompanying claims.

The liquid delivery pump intended to achieve the above-described object is a liquid delivery pump configured to deliver a drug while simulating a concentration of the delivered drug in a living body. The liquid delivery pump includes a calculation unit configured to calculate the concentration of the drug in the living body on the basis of the amount of delivered drug, a determination unit configured to determine whether one of a drug concentration of the drug in blood and an target site concentration of the drug, calculated by the calculation unit, has reached a predetermined target concentration, a storage unit configured to store an upper limit of the initial flow rate of the drug, an upper limit of an maintenance flow rate of the drug, and the target concentration, a reception unit configured to receive input of the target concentration, an upper limit value switching unit configured to switch an upper limit value of a delivery flow rate of the drug to one of the upper limit of the initial flow rate and the upper limit of the maintenance flow rate, according to a determination result from the determination unit, and an adjustment unit configured to adjust the delivery flow rate of the drug in a range that does not exceed the upper limit value.

### Advantageous Effects of Invention

According to the present invention, it is possible to calculate the concentration of a drug in the living body on the basis of the delivery flow rate of the drug. It is also possible to determine whether the calculated concentration of the drug in the living body has reached a predetermined target concentration. Furthermore, it is possible to switch the upper limit value of the delivery flow rate of the drug according to the determination result of whether the concentration of the drug in the living body has reached the predetermined target concentration. As a result, according to the present invention, it is possible, at delivery of a drug, to deliver the drug with an appropriate delivery flow rate that does not exceed a predetermined upper limit value prescribed according to whether efficacy of the drug is expressed for individual types of drugs.

Moreover, in a case where the upper limit of the initial flow rate is set as the upper limit value, it is possible to configure the upper limit value switching unit such that the upper limit value of the delivery flow rate at the time of delivery of drug can be switched from the upper limit of the initial flow rate to the upper limit of the maintenance flow rate when one of the target site concentration and the drug concentration in blood reaches the target concentration. This configuration makes it possible to deliver the drug at a delivery flow rate that does not exceed the allowable upper limit value of the delivery flow rate in a state where efficacy of the drug is expressed on a specific site of the living body.

Moreover, in a case where the upper limit of the maintenance flow rate is set as the upper limit value, it is possible to configure the upper limit value switching unit such that the upper limit value of the delivery flow rate at the time of delivery of the drug can be switched from the upper limit of the maintenance flow rate to the upper limit of the initial flow rate when one of the target site concentration and the drug concentration in a blood no longer satisfies the target concentration due to a change in the target concentration. This configuration makes it possible to promptly allow one of the target site concentration and the drug concentration in blood to reach the revised target concentration, and thus, to promptly allow efficacy of the drug to be expressed.

The adjustment unit may be configured to adjust the delivery flow rate at the time of delivery of the drug so as to maintain the drug concentration in blood at a target concentration. This configuration makes it possible to maintain the drug concentration in blood at a fixed level without exceeding the predetermined target concentration, and thus, to enhance safety.

The adjustment unit may be configured to adjust the delivery flow rate at the time of delivery of the drug so as to maintain the target site concentration at a target concentration. This configuration makes it possible to promptly allow the target site concentration to reach the target concentration, and thus, to allow the efficacy of the drug to be expressed more promptly.

Furthermore, the reception unit may be configured to limit reception of the input of the upper limit of the maintenance flow rate that exceeds the upper limit of the initial flow rate when storing the upper limit of the initial flow rate and the upper limit of the maintenance flow rate. This configuration makes it possible to prevent erroneously storing an upper limit of the maintenance flow rate that exceeds the upper limit of the initial flow rate, and thus, to enhance safety.

Moreover, the reception unit may be configured to receive input of a target concentration during liquid delivery. This configuration makes it possible to change the target concentration according to situation, and thus, to further enhance safety and convenience.

Furthermore, the upper limit value switching unit may be configured to switch the upper limit value of the delivery flow rate in a case where one of the target site concentration and the drug concentration in blood is included within a predetermined tolerance range based on the target concentration. This configuration makes it possible to flexibly set a timing to switch the upper limit value of the delivery flow rate according to the situation, and thus, to further enhance convenience.

Moreover, it is possible to provide a reporting unit configured to report that the upper limit value of the delivery flow rate of the drug is switched. This configuration makes it possible to grasp switching of the upper limit value of the delivery flow rate of the drug at an appropriate timing, and thus, to further enhance safety.

### Brief Description of Drawings

Fig. 1 is a schematic perspective view for illustrating a syringe pump according to a first embodiment of the present invention.
Fig. 2 is a schematic front view for illustrating the syringe pump according to the first embodiment of the present invention.
Fig. 3 is a schematic front view for illustrating a display unit of the syringe pump according to the first embodiment of the present invention.
Figs. 4(A) and 4(B) are schematic front views for illustrating the display unit of the syringe pump according to the first embodiment of the present invention, representing an exemplary display for inputting information on a patient to whom a drug is to be delivered.
Fig. 5 is a schematic front view for illustrating the display unit of the syringe pump according to the first embodiment of the present invention, representing an exemplary display for inputting the type of drug to be delivered.
Fig. 6 is a schematic front view for illustrating the display unit of the syringe pump according to the first embodiment of the present invention, representing an exemplary display for inputting a target concentration.
Fig. 7 is a schematic perspective view for illustrating a syringe used in the syringe pump according to the first embodiment of the present invention.
Fig. 8 is a schematic view for illustrating an electrical configuration of the syringe pump according to the first embodiment of the present invention.
Fig. 9 is a diagram for illustrating operation of a control unit of the syringe pump according to the first embodiment of the present invention, represented by a flowchart for illustrating operation related to switching of the upper limit value of the delivery flow rate and related to adjustment of the delivery flow rate.
Fig. 10 is a diagram illustrating a change in the drug concentration in blood and the target site concentration, of the delivered drug, representing a case where liquid delivery is performed using the syringe pump according to the first embodiment of the present invention.
Figs. 11(A) to 11(C) are external front view for illustrating the display unit of the syringe pump according to the first embodiment of the present invention, representing ongoing liquid delivery using the syringe pump.
Fig. 12 is an external front view for illustrating an exemplary display of a display unit according to a modification of the first embodiment of the present invention, representing an exemplary display when the target concentration and its tolerance are input.
Fig. 13 is a diagram for illustrating operation of a control unit of a syringe pump according to a second embodiment of the present invention, represented by a flowchart illustrating operation related to switching of the upper limit value of the delivery flow rate and related to adjustment of the delivery flow rate.
Fig. 14 is a diagram illustrating a change in the drug concentration in blood and the target site concentration, of the delivered drug, representing a case where liquid delivery is performed using the syringe pump according to the second embodiment of the present invention.
Figs. 15(A) and 15(B) are external front views for illustrating an exemplary display on the display unit according to a modification of an embodiment of the present invention, representing an exemplary display for selecting the concentration to be used as a reference for switching the upper limit value, and for selecting the concentration to be maintained at a target concentration.

### Description of Embodiments

### <First Embodiment>

Hereinafter, a first embodiment of the present invention will be described with reference to the drawings. For the purpose of explanation, proportions of dimensions in the drawings may be exaggerated and may differ from the proportions in reality in some cases.

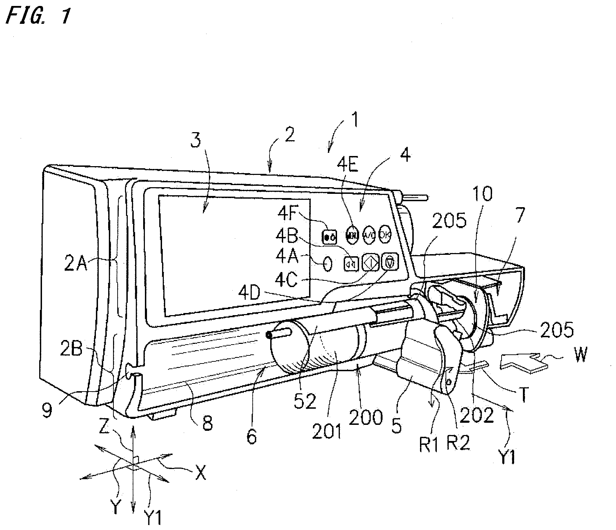

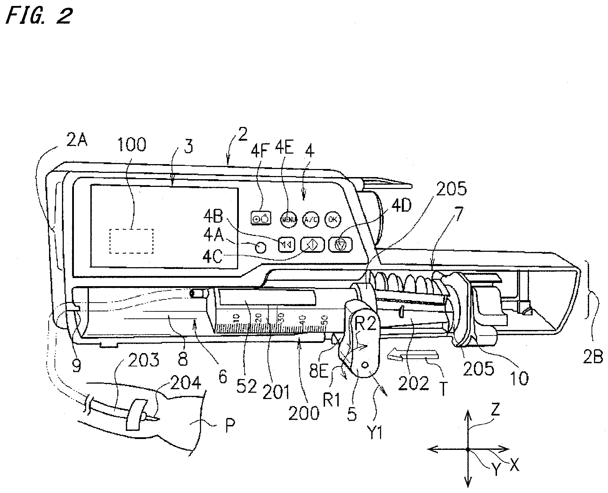

A syringe pump 1 according to a first embodiment of the present invention, illustrated in Figs. 1 and 2, is a liquid delivery pump, for example, used for delivering a drug into the body of a patient for a long time in an intensive care unit including an ICU, a CCU, and an NICU.

The syringe pump 1 is capable of delivering various types of drugs including intravenous anesthetic into the body of the patient. Exemplary applicable intravenous anesthetic includes propofol, midazolam, and remifentanil.

Typically, the delivery flow rate at the time of delivery of a drug into the body of a patient has its upper limit value determined for individual types of drugs. The delivery flow rate represents the flow velocity using a unit of ml/kg/second, and ml/kg/hour, for example. The upper limit value of the delivery flow rate at introduction until efficacy of the delivered drug is expressed (hereinafter, upper limit of an initial flow rate) differs from the upper limit value of the delivery flow rate for maintaining efficacy of the drug after expression of the efficacy of the drug (hereinafter, upper limit of a maintenance flow rate). Typically, the upper limit of the maintenance flow rate is smaller than the upper limit of the initial flow rate. This is because it would be dangerous to deliver the drug with a delivery flow rate exceeding the upper limit of the maintenance flow rate even when the efficacy of the drug is expressed. A document describing usage and precautions of the drug (hereinafter, drug package insert) is attached to each of the drugs. The upper limit of the initial flow rate and the upper limit of the maintenance flow rate are described in the drug package insert. In some cases, an expression such as a proper value as the delivery flow rate instead of the upper limit value of the delivery flow rate is used for description depending on the drug, but they are substantially the same. Delivery of the drug needs to be performed at an appropriate delivery flow rate that does not exceed the upper limit of the initial flow rate or the upper limit of the maintenance flow rate, according to whether the efficacy of the drug has been expressed.

To achieve this, the syringe pump 1 delivers the drug at an appropriate delivery flow rate by switching the upper limit value of the delivery flow rate to one of the upper limit of the initial flow rate and the upper limit of the maintenance flow rate, determined for individual types of drugs, according to whether the efficacy of the drug is expressed.

Hereinafter, an apparatus configuration of the syringe pump 1 will be described in detail.

As illustrated in Figs. 1 and 2, the syringe pump 1 correctly delivers the drug inside a syringe main body 201 by pressing, in a T-direction, a syringe plunger 202 of a syringe 200 as a drug container in which the drug is filled, to the patient, via a tube 203 and an indwelling needle 204. At this time, the syringe main body 201 of the syringe 200 is fixed to the syringe pump 1 with a clamp 5 so as not to move.

The syringe pump 1 includes a main body cover 2.

The main body cover 2 is integrally molded with a molding resin material having chemical resistance. Accordingly, the main body cover 2 has a splash-proof treatment structure. With this splash-proof treatment structure, even when a drug, or the like, is attached, it is possible to prevent it from entering the inner portion of the syringe pump 1. This splash-proof treatment structure is provided against a case where the drug inside the syringe main body 201 spills, infusion fluid arranged above drops down, or antiseptic solution, or the like, used in surrounding areas scatters and attaches to the pump.

As illustrated in Figs. 1 and 2, the main body cover 2 includes an upper portion 2A and a lower portion 2B.

A display unit 3 and an operation panel unit 4 are arranged on the upper portion 2A.

A syringe setting unit 6 and a syringe plunger drive unit 7 are arranged on the lower portion 2B. The syringe plunger drive unit 7 is provided for pressing the syringe plunger 202.

The display unit 3 is an image display apparatus capable of displaying color images. The display unit 3 may be configured with a color liquid crystal display apparatus, for example. The display unit 3 is capable of displaying information not only in Japanese but also in a plurality of foreign languages, as required. The display unit 3 is arranged at an upper-left position of the upper portion 2A of the main body cover 2, above the syringe setting unit 6 and the syringe plunger drive unit 7.

The operation panel unit 4 is arranged on the right side of the display unit 3, on an upper portion of the main body cover 2. On the operation panel unit 4, a power ON/OFF button 4F, an operation indicator 4A, and operation buttons are arranged. Figs. 1 and 2 illustrate an example in which four minimum buttons, namely, a fast delivery switch button 4B, a start switch button 4C, a stop switch button 4D, and a menu selection button 4E, are arranged as operation buttons.

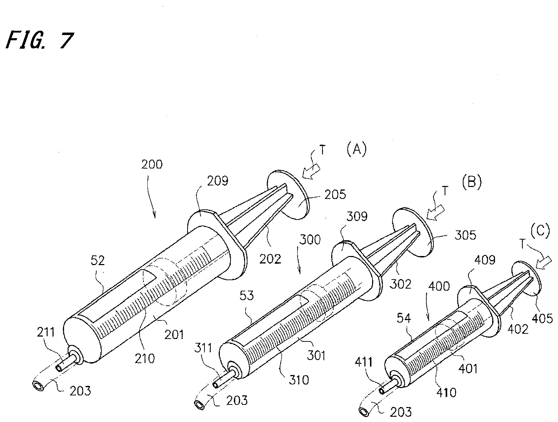

As illustrated in Figs. 1 and 2, the syringe setting unit 6 and the syringe plunger drive unit 7 are arranged side by side in the X-direction. A plurality of different-sized syringes 200, 300, and 400 is selectably and removably inserted and fixed into the syringe setting unit 6. The syringes will be described below with reference to Fig. 7.

As illustrated in Figs. 1 and 2, the syringe setting unit 6 includes the container 8 and the clamp 5. The container 8 contains the syringe main body 201. The container 8 is a recess having a substantially a semicircular cross section in order to contain the syringe main body 201 and is formed in the X-direction. A tube fixing section 9 is provided on a wall portion on an end of the container 8. The tube fixing section 9 is provided to removably hold the tube 203.

When operating the clamp 5 to remove the syringe 200 from the syringe setting unit 6, by pulling the clamp 5 in a Y1 direction (front direction) against the force of a spring (not illustrated) and by rotating the clamp 5 for 90 degrees in an R1 direction, it is possible to remove the syringe main body 201 from the container 8, being released from a state of being fixed by the clamp 5. When operating the clamp 5 to mount the syringe 200 onto the syringe setting unit 6, by pulling the clamp 5 in the Y1 direction against the force of the spring (not illustrated) and by rotating the clamp 5 for 90 degrees in an R2 direction, and by returning the clamp 5 in a Y2 direction by the force of the spring, it is possible to contain the syringe main body 201 into the container 8 to be fixed by the clamp 5. On the syringe setting unit 6, a right end portion 8E of the container 8 includes a notch portion such that the clamp 5 can fix syringes having capacities 2.5 mL, 5 mL, 10 mL, 20 mL, 30 mL, and 50 mL.

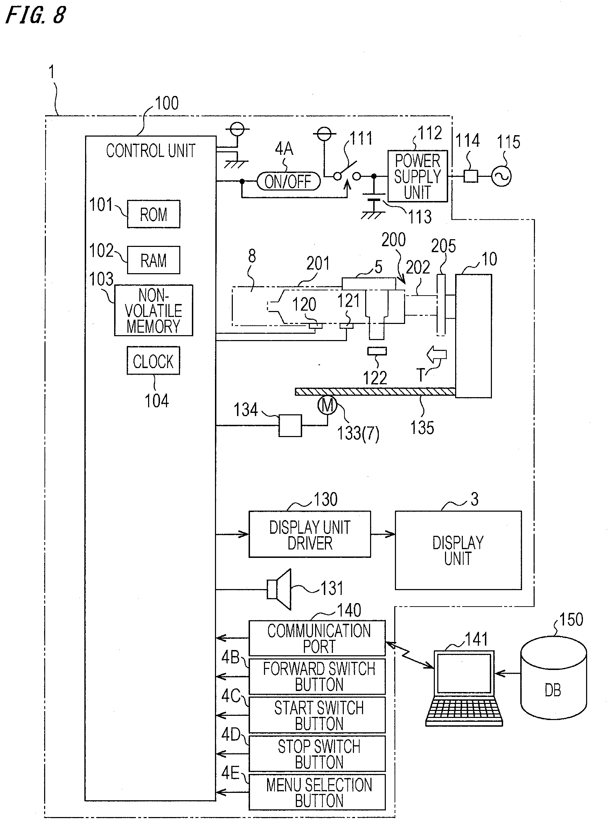

When the syringe main body 201 is contained and fixed in the container 8, the syringe plunger 202 is arranged inside the syringe plunger drive unit 7. The syringe plunger drive unit 7 includes a slider 10. According to an instruction from a control unit 100 illustrated in Figs. 2 and 8, the slider 10 gradually presses a plunger flange 205 of the syringe plunger 202 along the T-direction relative to the syringe main body 201.

The X-direction, the Y-direction, and the Z-direction in Figs. 1 and 2 are orthogonal to each other. The Z-direction represents an up-down direction.

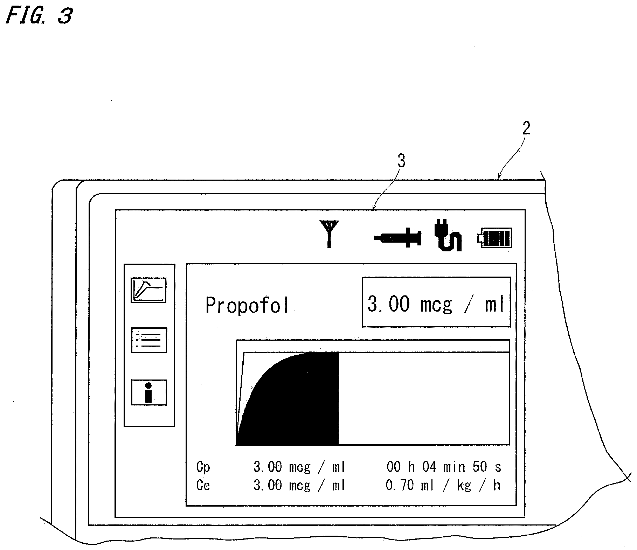

Fig. 3 illustrates exemplary displayed information on the display unit 3. The exemplary display on the display unit 3 is only an example, and not limited to this, in particular.

Fig. 7 is a perspective view illustrating an exemplary plurality of types of syringes described above.

Each of Figs. 1 and 2 illustrates an example in which the syringe 200 having a largest drug capacity is fixed.

As illustrated in Fig. 7(A), the syringe 200 having the largest drug capacity includes the syringe main body 201 and the syringe plunger 202. The syringe main body 201 includes a main-body flange 209. The syringe plunger 202 includes the plunger flange 205. The syringe main body 201 includes a drug scale 210. An end portion of the flexible tube 203 is removably connected to an outlet 211 of the syringe main body 201.

As illustrated in Fig. 7(B), the syringe 300 having a medium-sized drug capacity includes a syringe main body 301 and a syringe plunger 302. The syringe main body 301 includes a main-body flange 309. The syringe plunger 302 includes a plunger flange 305. The syringe main body 301 includes a drug scale 310. An end portion of the flexible tube 203 is removably connected to an outlet 311 of the syringe main body 301.

As illustrated in Fig. 7(C), the syringe 400 having a smallest drug capacity includes a syringe main body 401 and a syringe plunger 402. The syringe main body 401 includes a main-body flange 409. The syringe plunger 402 includes a plunger flange 405. The syringe main body 401 includes a drug scale 410. An end portion of the flexible tube 203 is removably connected to an outlet 411 of the syringe main body 401.

The exemplary drug capacity of the syringe 200 illustrated in Fig. 7(A) may be 50 mL, the exemplary drug capacity of the syringe 300 illustrated in Fig. 7(B) may be 10 mL, 20 mL, and 30 mL, and the exemplary drug capacity of the syringe 400 illustrated in Fig. 7(C) may be 2.5 mL and 5 mL. The syringes 300 and 400 may be contained and fixed inside the container 8 when in use similarly to the case of the syringe 200 illustrated in Figs. 1 and 2.

Next, an electrical configuration of the syringe pump 1 illustrated in Figs. 1 and 2 will be described in detail with reference to Fig. 8.

In Fig. 8, the syringe pump 1 includes the control unit (computer) 100 configured to control and judgment of overall operation. The control unit 100 is, for example, a one-chip microcomputer, and includes a read only memory (ROM) 101, a random access memory (RAM) 102, a non-volatile memory 103, and a clock 104.

With predetermined operation, the clock 104 is capable of correcting the current time, acquiring the current time, measuring elapsed time of predetermined liquid delivery operation, measuring the reference time for liquid delivery speed control, or the like.

The control unit 100 illustrated in Fig. 8 is connected with a power ON/OFF button 4F and a switch 111.

The switch 111 supplies power to the control unit 100 from any of a power supply converter unit 112 and a storage battery 113 such as a lithium ion battery by switching between the power supply converter unit 112 and the storage battery 113.

The power supply converter unit 112 is connected to a commercial AC power supply 115 via a receptacle 114.

In Fig. 8, a pair of detection switches 120 and 121 is arranged inside the container 8. Each of the detection switches 120 and 121 detects whether the syringe main body 201 of the syringe 200 is properly arranged in the container 8 and reports the result to the control unit 100.

A clamp sensor 122 detects a positional state of the clamp 5 and thereby reporting whether the syringe main body 201 is securely clamped by the clamp 5, to the control unit 100.

When a motor 133 of the syringe plunger drive unit 7 is driven by a motor driver 134 according to an instruction from the control unit 100, the motor 133 moves the slider 10 in the T-direction by rotating a feed screw 135. Subsequently, by pressing the syringe plunger 202 in the T-direction, the slider 10 correctly delivers the drug contained in the syringe main body 201 illustrated in Fig. 2 to a patient P through the tube 203 via the indwelling needle 204.

In Fig. 8, the fast delivery switch button 4B, the start switch button 4C, the stop switch button 4D, and the menu selection button 4E are electrically connected to the control unit 100. When the start switch button 4C is pressed, a control signal for starting liquid delivery is input into the control unit 100. When the stop switch button 4D is pressed, a control signal for stopping liquid delivery is input into the control unit 100.

In Fig. 8, a display unit driver 130 is electrically connected to the control unit 100. According to an instruction from the control unit 100, the display unit driver 130 drives the display unit 3 to display various information on the display unit 3.

In Fig. 8, a speaker 131 is electrically connected to the control unit 100. According to an instruction from the control unit 100, the speaker 131 performs announcement of various alarms with voice.

The control unit 100 has a function as a calculation unit to calculate the concentration of the drug inside a living body on the basis of the amount of drug delivered into the living body from the start of delivery up to the current time.

The amount of drug delivered into the living body from the start of delivery can be calculated, for example, by multiplying the inner diameter of the syringe main body 201 of the syringe 200 and the amount of movement of the slider 10 in the T-direction by the feed screw 135 from the start of delivery.

The concentration of the drug in the living body is calculated by simulation.

The simulation is performed using three-compartment model, on the basis of pharmacokinetics, but not limited to this.

The simulation using the three-compartment model calculates concentrations by dividing the internal portion of the body into three portions (hereinafter, compartments). One of the three compartments is a compartment that has modeled blood. The other two compartments have modeled a biological tissue having abundant blood flow, such as muscle, and a biological tissue having less blood flow, such as fat, respectively. The drug is administered to a compartment that has modeled blood. The drug moves between the compartment that has modeled blood and the other two compartments at a predetermined moving speed. The drug is discharged from the body at a predetermined discharge speed via the compartment that has modeled blood. The concentration, including the drug concentration in blood, of the delivered drug in individual compartments can be calculated on the basis of information on a patient to whom the drug is delivered, and the relationships among the amount of delivered drug, the moving speed, the discharge speed, or the like. By including a compartment that has modeled a site to which the drug is applied, into the compartment, it is also possible to calculate the target site concentration, namely, the concentration of the site to which the drug is applied. Exemplary target sites would include the brain for sedative (propofol, or the like), and the neuromuscular junctions for muscle relaxant (midazolam, or the like). The delivery flow rate is calculated on the basis of a difference between one of the calculated drug concentration of the drug in blood and the target site concentration of the drug, calculated in this manner, and the set target concentration. In a case where the delivery flow rate exceeds the upper limit value, the delivery flow rate is set to the upper limit value.

The non-volatile memory 103 stores the upper limit of the initial flow rate and the upper limit of the maintenance flow rate for individual types of drugs. The non-volatile memory 103 also stores a target concentration. The target concentration is stored in a unit of mcg/ml, for example. The non-volatile memory 103 stores information of the patient to whom the drug is delivered, and the type of drug to be delivered. Information of the patient to be stored includes, for example, sex, age, height, and weight. As described below, the non-volatile memory 103 stores the upper limit value of the delivery flow rate to be switched by the control unit 100.

The control unit 100 has a function as a reception unit to receive input of information such as the upper limit of the initial flow rate, the upper limit of the maintenance flow rate, the target concentration, patient information, and the types of delivered drug, to be stored in the non-volatile memory 103.

The control unit 100 stores information for which input has been received, into the non-volatile memory 103.

The control unit 100 receives input even during delivery of the drug. Accordingly, the target concentration stored in the non-volatile memory 103 can be changed during delivery of the drug.

The control unit 100 checks the input upper limit of the initial flow rate and the upper limit of the maintenance flow rate and limits reception of invalid input. Specifically, the control unit 100 checks a difference between the upper limit of the initial flow rate and the upper limit of the maintenance flow rate. In a case where the upper limit of the maintenance flow rate exceeds the upper limit of the initial flow rate, the input upper limit of the initial flow rate and the upper limit of the maintenance flow rate are not stored in the non-volatile memory 103.

Input of information regarding the upper limit of the initial flow rate and the upper limit of the maintenance flow rate into the control unit 100 can be performed with various methods.

For example, the upper limit of the initial flow rate and the upper limit of the maintenance flow rate for individual types of drugs can be input by operating an operation button on the operation panel unit 4 connected to the control unit 100.

Input is also possible via a communication port 140. For example, as illustrated in Fig. 8, a computer 141 including a desktop computer is connected with the control unit 100 via the communication port 140. By operating the computer 141, the upper limit of the initial flow rate and the upper limit of the maintenance flow rate can be input into the control unit 100 for individual types of drugs via the communication port 140.

In this case, the computer 141 may be connected to a drug database 150 as illustrated in Fig. 8. The drug database 150 can store the upper limit of the initial flow rate and the upper limit of the maintenance flow rate for individual types of drugs, collectively as a drug library. By operating the computer 141, the upper limit of the initial flow rate and the upper limit of the maintenance flow rate stored in the drug database 150 for individual types of drugs can be input into the control unit 100 via the communication port 140. The drug library may record information regarding the drug, other than the information of the upper limit of the initial flow rate and the upper limit of the maintenance flow rate. For example, the drug library may record a drug manufacturer and contraindicated information for individual types of drugs. The information can be input into the control unit 100 together with the upper limit of the initial flow rate and the upper limit of the maintenance flow rate, via the communication port 140. The drug library may be collectively generated for overall hospital or each of hospital wards, and stored in the drug database 150.

Information on the patient to whom liquid delivery is performed, the type of drug to be delivered, and the target concentration can be input into the control unit 100 by operating the operation button on the operation panel unit 4 according to the displayed information on the display unit 3.

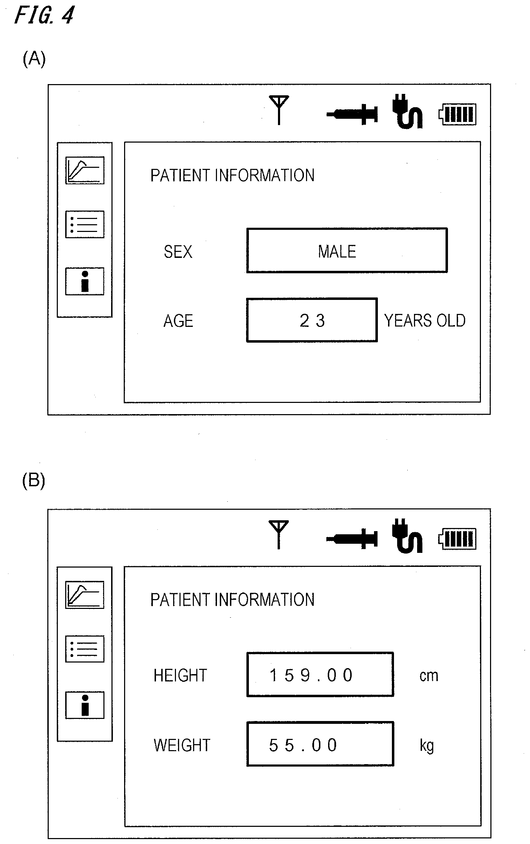

Figs. 4(A) and 4(B) illustrate exemplary displayed information of the display unit 3 when the information on a patient to whom liquid delivery is performed is input. By operating the operation button on the operation panel unit 4 according to the exemplary displayed information illustrated in Figs. 4(A) and 4(B), it is possible to input information on the patient for liquid delivery, such as sex, age, height, and weight.

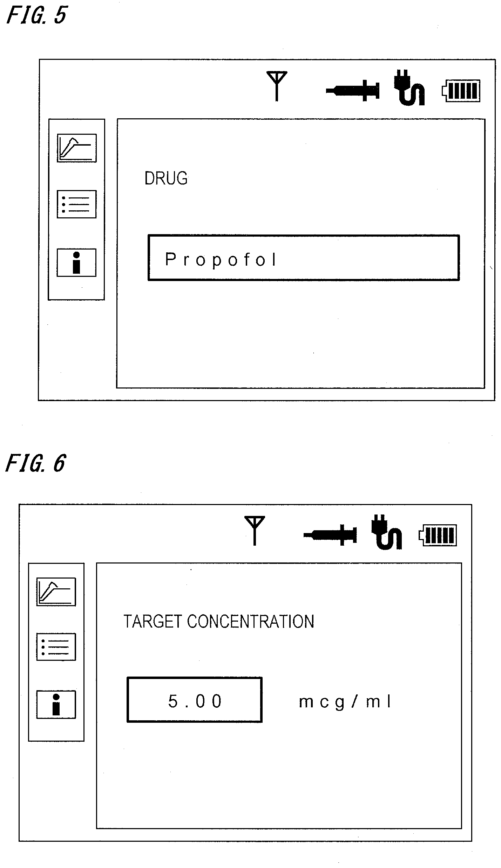

Fig. 5 illustrates exemplary displayed information on the display unit 3 when the type of drug to be delivered is input. By operating the operation button on the operation panel unit 4 according to the exemplary displayed information illustrated in Fig. 5, it is possible to input the type of drug to be delivered.

Fig. 6 is exemplary displayed information on the display unit 3 when the target concentration is input. By operating the operation button on the operation panel unit 4 according to the exemplary displayed information illustrated in Fig. 6, it is possible to input the target concentration.

The control unit 100 starts delivering the liquid when the start switch button 4C is pressed and the control signal for start delivery is input. The control unit 100 stops delivery of the liquid when the stop switch button 4D is pressed and the control signal for stop is input.

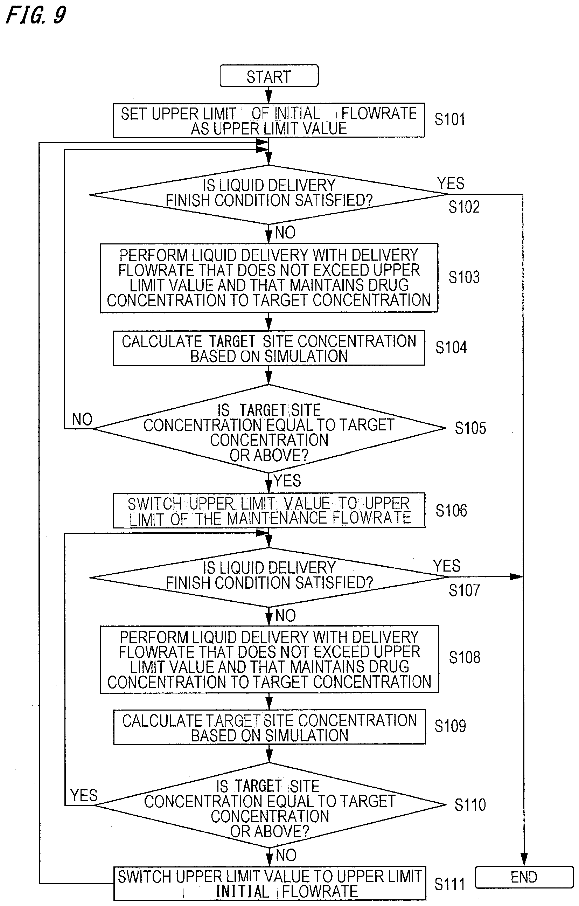

The control unit 100 has functions as a determination unit configured to determine whether the target site concentration of the delivered drug has reached the target concentration, as an upper limit value switching unit configured to switch upper limit values of the delivery flow rate, and as an adjustment unit configured to adjust the delivery flow rate. Hereinafter, operation of these functions will be described with reference to the flowchart illustrated in Fig. 9.

First, step S101 sets the upper limit of the initial flow rate stored in the non-volatile memory 103 as an upper limit value of the delivery flow rate of the drug and stores the value in the non-volatile memory 103 according to the type of drug to be delivered, stored in the non-volatile memory 103.

Next, step S102 determines whether the liquid delivery finish condition is satisfied. If the liquid delivery finish condition is satisfied, the liquid delivery is finished. The liquid delivery finish condition would be satisfied at least in a case where the stop switch button 4D is pressed and the control signal of liquid delivery finish has been received.

Nest, step S103 performs liquid delivery with a delivery flow rate that does not exceed the upper limit value stored in the non-volatile memory 103 and that maintains the drug concentration of the drug in blood after delivery at the target concentration stored in the non-volatile memory 103 The drug concentration of the drug in blood after delivery can be calculated by simulation on the basis of the amount of delivered drug, patient information and the type of drug to be delivered, stored in the non-volatile memory 103. Delivery of the drug is performed by the slider 10 when it presses the syringe plunger 202 in the T-direction (refer to Figs. 1 and 2). The slider 10 is moved by the drive of the motor driver 134 of the syringe plunger drive unit 7 (refer to Fig. 8).

Next, step S104 calculates the target site concentration of the delivered drug by simulation on the basis of the amount of drug delivered in step S103, patient information and the type of drug to be delivered, stored in the non-volatile memory 103.

Next, step S105 determines whether the target site concentration calculated in step S104 has reached the target concentration stored in the non-volatile memory 103. In a case where it is determined that the target concentration has not been reached, processing returns to step S102. In a case where it is determined that the target concentration has been reached, processing moves on to step S106.

Step S106 switches the upper limit value of the delivery flow rate from the upper limit of the initial flow rate to the upper limit of the maintenance flow rate. Switching of the upper limit value is performed by rewriting the upper limit value stored in the non-volatile memory 103. That is, switching of the upper limit value is performed by setting the upper limit of the maintenance flow rate stored in the non-volatile memory 103 as the upper limit value of the delivery flow rate of the drug and by storing the value in the non-volatile memory 103 according to the type of drug to be delivered, stored in the non-volatile memory 103.

After switching of the upper limit value, the control unit 100 reports that the upper limit value has been switched. There are a variety of methods for reporting. For example, switching of the upper limit value can be reported with a buzzer sound from the speaker 131 in response to an instruction issued to the speaker 131. Switching of the upper limit value can also be reported with texts and images displayed on the display unit 3 to indicate the switching in response to an instruction issued to the display unit driver 130.

Next, similarly to step S102, step S107 determines whether the liquid delivery finish condition is satisfied. If the liquid delivery finish condition is satisfied, the liquid delivery is finished.

Nest, step S108 performs liquid delivery with the delivery flow rate that does not exceed the upper limit value stored in the non-volatile memory 103 and that maintains the drug concentration in blood, after delivery, of the drug at the target concentration stored in the non-volatile memory 103. Similarly to step S104, the drug concentration in blood, after delivery, of the drug can be calculated by simulation. Delivery of the drug is performed by pressing of the syringe plunger 202, similarly to step S104.

Next, step S109 calculates the target site concentration of the delivered drug by simulation on the basis of the amount of drug delivered in step S108, patient information and the type of drug to be delivered stored in the non-volatile memory 103, or the like.

Next, step S110 determines whether the target site concentration calculated in step S109 has reached the target concentration stored in the non-volatile memory 103. In a case where it is determined that the target concentration has been reached, processing returns to step S107. In a case where it is determined that the target concentration has not been reached, processing moves on to step S111.

Step S111 switches the upper limit value of the delivery flow rate from the upper limit of the maintenance flow rate to the upper limit of the initial flow rate. That is, the upper limit of the initial flow rate stored in the non-volatile memory 103 is set as an upper limit value of the delivery flow rate of the drug and the value is stored in the non-volatile memory 103 according to the type of drug to be delivered, stored in the non-volatile memory 103. After switching of the upper limit value, the control unit 100 reports that the upper limit value has been switched, and returns to step S102.

Hereafter, the above-described steps are repeated until the liquid delivery finish condition is satisfied.

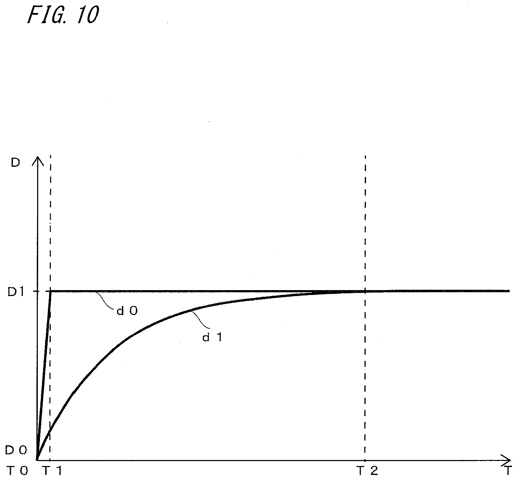

Fig. 10 is a graph illustrating an exemplary concentration change in the drug in the living body when the drug is delivered using the syringe pump 1. In this, d0 represents a drug concentration in blood, d1 represents an target site concentration, and D1 represents a target concentration. T0 represents delivery start time, T1 represents the time at which the drug concentration in blood reaches the target concentration D1, and T2 represents the time at which the target site concentration reaches the target concentration D1.

At time T0, the syringe pump 1 starts delivery of the drug while setting the upper limit of the initial flow rate as the upper limit value of the delivery flow rate. After starting liquid delivery, the drug concentration in blood d0 reaches the target concentration D1 at time T1. On and after time T1, the drug concentration in blood d0 is maintained at the target concentration. When the liquid delivery is continued, the target site concentration d1 reaches the target concentration D1. At this time, the upper limit value of the delivery flow rate is switched from the upper limit of the initial flow rate to the upper limit of the maintenance flow rate.

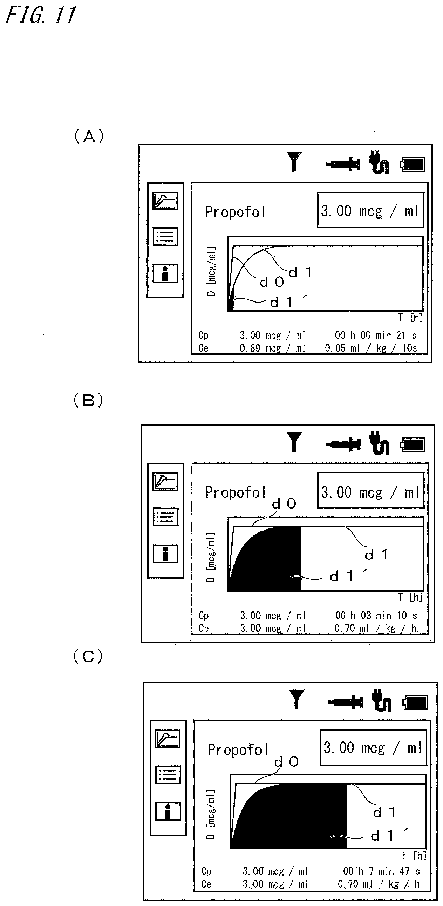

Figs. 11(A) to 11(C) are exemplary transition of displayed information on the display unit 3 during drug delivery. Fig. 11(A) illustrates an exemplary display of the display unit 3 immediately after starting drug delivery. Fig. 11(B) illustrates an exemplary display of the display unit 3 when the target site concentration d1 reaches the target concentration. Fig. 11(C) illustrates an exemplary display of the display unit 3 after the target site concentration d1 reaches the target concentration.

As illustrated in Figs. 11(A) to 11(C), the display unit 3 displays, during drug delivery, an estimated line for the drug concentration of the drug in blood d0 and an estimated line for the target site concentration d1 of the drug, calculated on the basis of simulation. An axis T represents time and an axis D represents concentration. A solid black region d1' represents history of transition of the target site concentration d1, from the start of liquid delivery up to the current point.

Next, exemplary usage of the syringe pump 1 will be described.

First, as illustrated in Fig. 8, the control unit 100 is connected with the computer 141 via the communication port 140.

Next, with operation on the computer 141, drug library information stored in the drug database 150 is input into the control unit 100 via the communication port 140. With this operation, the upper limit of the initial flow rate and the upper limit of the maintenance flow rate recorded in the drug library are stored in the non-volatile memory 103 for individual types of drugs.

Next, as illustrated in Figs. 1 and 2, the syringe 200 is set onto the syringe pump 1. Setting of the syringe pump 1 is performed with an above-described method using the clamp 5. Subsequently, the indwelling needle 204 to which the tube 203 is connected is inserted into the patient.

Next, as illustrated in Figs. 4(A) to 6, various information is input using the display unit 3 and the operation panel unit 4. First, as information of the patient, sex, age, height, and weight are input. Next, the type of drug to be delivered is input. Then, the target concentration is input.

Next, delivery of the drug into the body of the patient is started by pressing the start switch button 4C. Switching of the upper limit value of the delivery flow rate and adjustment of the delivery flow rate are performed by the control unit 100 according to the flowchart illustrated in Fig. 9. Liquid delivery is performed until the above-described liquid delivery finish condition is satisfied.

When the upper limit value of the delivery flow rate is switched, the speaker 131 issues a buzzer sound to report the switching of the upper limit value.

It is also possible to change the target concentration during liquid delivery. Specifically, it is possible to change the target concentration in a case where drug efficacy is not expressed even though the target site concentration has reached the target concentration. Alternatively, it is also possible to change the target concentration in a case where the drug efficacy is expressed before the target site concentration reaches the target concentration. The target concentration can be changed, as described above, by operating on the operation panel unit 4 according to the display on the display unit 3.

According to the present embodiment, it is possible to calculate the concentration of a drug in the living body on the basis of the delivery flow rate of the drug. It is also possible to determine whether the calculated concentration of the drug in the living body has reached a predetermined target concentration. Furthermore, it is possible to switch the upper limit value of the delivery flow rate of the drug according to the determination result of whether the concentration of the drug in the living body has reached the predetermined target concentration. As a result, according to the present embodiment, it is possible, at delivery of a drug, to deliver the drug with an appropriate delivery flow rate that does not exceed a predetermined upper limit value prescribed for individual types of drugs.

Moreover, according to the present embodiment, in a case where the upper limit of the initial flow rate is set as the upper limit value, it is possible to switch the upper limit value of the delivery flow rate at the time of delivery of the drug, from the upper limit of the initial flow rate to the upper limit of the maintenance flow rate when the target site concentration calculated by simulation reaches the target concentration. This makes it possible to deliver a drug at a delivery flow rate that does not exceed the allowable upper limit of the maintenance flow rate in a state where the efficacy of the drug is expressed on a specific site of the living body.

Moreover, according to the present embodiment, in a case where the upper limit of the maintenance flow rate is set as the upper limit value, it is possible to switch the upper limit value of the delivery flow rate at the time of delivery of the drug, from the upper limit of the maintenance flow rate to the upper limit of the initial flow rate, when the target site concentration no longer satisfies the target concentration due to a change in the target concentration. This makes it possible to promptly allow one of the target site concentration and the drug concentration in blood to reach the revised target concentration, and thus, to allow the efficacy of the drug to be expressed.

According to the present embodiment, it is also possible to adjust the delivery flow rate at the time of delivery of the drug to maintain the drug concentration in blood at a target concentration. This makes it possible to maintain the drug concentration in blood at a fixed level without exceeding the predetermined target concentration, leading to enhanced safety.

Furthermore, according to the present embodiment, reception of the input of the upper limit of the maintenance flow rate that exceeds the upper limit of the initial flow rate is limited when the upper limit of the initial flow rate and the upper limit of the maintenance flow rate are going to be stored. This makes it possible to prevent erroneously storing an upper limit of the maintenance flow rate that exceeds the upper limit of the initial flow rate, and thus, to enhance safety.

According to the present embodiment, it is possible to receive the input of the target concentration during liquid delivery. This makes it possible to change the target concentration according to situation, and thus, to further enhance safety and convenience.

According to the present embodiment, it is possible to report that the upper limit value of the delivery flow rate of the drug is switched. This makes it possible to grasp switching of the upper limit value of the delivery flow rate of the drug at an appropriate timing, and thus, to further enhance safety.

### <Modification>

In the above-described embodiment, the control unit 100 switches the upper limit value according to whether the simulated target site concentration has reached the target concentration. Alternatively, it is allowable to set the target site concentration for which the upper limit value is going to be switched, to a different level from the target concentration. The present modification will describe a syringe pump configured such that the upper limit value is switched according to whether the target site concentration is included in a predetermined tolerance range on the basis of the target concentration.

In the syringe pump according to the present modification, the control unit 100 switches the upper limit value of the delivery flow rate according to the following condition. That is, for example, in a case where the target concentration is 3.00 mcg/ml, and a tolerance is ±5.00%, the upper limit value of the delivery flow rate is switched according to whether the target site concentration is included in a range from a level (3.00 - 3.00 × 0.05) mcg/ml or above and below a level (3.00 + 3.00 × 0.05) mcg/ml.

Similarly to the target concentration, the tolerance can also be input into the control unit 100 by operating the operation buttons on the operation panel unit 4 according to the displayed information on the display unit 3. The input tolerance is stored in the non-volatile memory 103.

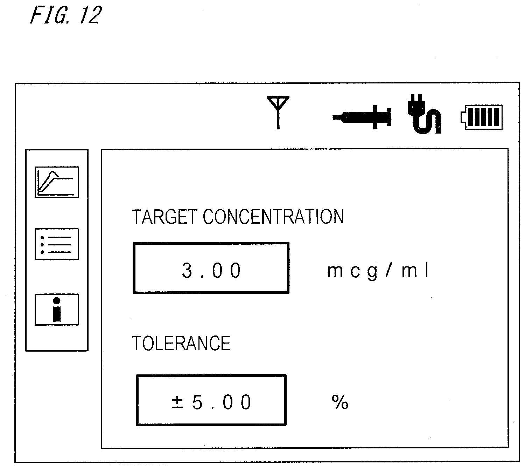

Fig. 12 illustrates an exemplary display of the display unit 3 in a case of inputting the tolerance together with the target concentration. Fig. 12 illustrates an exemplary case where the target concentration of 3.00 mcg/ml and the tolerance of ±5.00% are used for input.

With the above-configured control unit 100, it is possible to switch the upper limit value of the delivery flow rate in a case where the target site concentration is included in a tolerance range based on the target concentration. This makes it possible to flexibly set the timing to switch the upper limit value of the delivery flow rate according to the situation, and thus, to further enhance convenience.

In the above-described embodiment and modification, the control unit 100 switches the upper limit value when the simulated target site concentration reaches the target concentration, but the configuration is not limited to this. For example, the control unit 100 may be configured such that the upper limit value is switched when the simulation drug concentration in blood reaches the target concentration. With the control unit 100 being configured in this manner, it is possible to deliver the drug while adjusting the delivery flow rate more appropriately according to an applied technique and the types of drugs.

### <Second Embodiment>

Next, a syringe pump according to a second embodiment of the present invention will be described.

In the syringe pump according to the second embodiment, operation related to switching of the upper limit value of the delivery flow rate and the operation related to adjustment of the delivery flow rate, performed by the control unit, differ from the operation of the control unit 100 of the syringe pump according to the first embodiment. The control unit 100 of the syringe pump 1 according to the first embodiment adjusts the delivery flow rate such that the drug concentration in blood can be maintained at a target concentration. The control unit of the syringe pump according to the second embodiment differs from the control unit 100 in that the delivery flow rate is adjusted such that the target site concentration can be maintained at a target concentration. For units other than the control unit, namely, for the main body cover 2, the display unit 3, the operation panel unit 4, or the like, the configuration is same as the configuration in the first embodiment, and thus, description will be omitted. Among the operation of the control unit of the syringe pump according to the second embodiment, operation of the motor driver 134, operation of reception of input of target concentration, or the like, are the same as the operation in the first embodiment, and thus, description will be omitted.

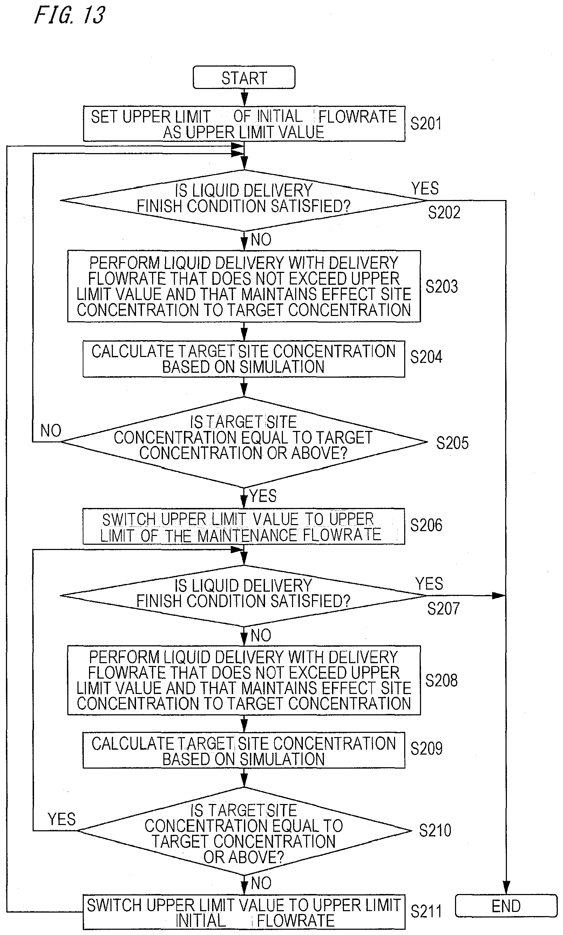

Fig. 13 is a flowchart illustrating switching of the upper limit value of the delivery flow rate and operation related to adjustment of the delivery flow rate, performed by the control unit of the syringe pump according to the second embodiment.

A portion of operation of the control unit of the syringe pump according to the second embodiment, illustrated in Fig. 13, is the same as a portion of the operation of the control unit 100 of the syringe pump 1 according to the first embodiment. Specifically, step S201 for setting the upper limit of the initial flow rate corresponds to step S101. Furthermore, step S202 and step S207 for determining whether the liquid delivery finish condition is satisfied correspond to step S102 and S107, respectively. Furthermore, step S206 and S211 for switching the upper limit value correspond to step S106 and step S111, respectively. Description will be omitted for these steps, and hereinafter, steps having difference will be described.

First, step S203 performs liquid delivery with a delivery flow rate that does not exceed the upper limit value stored in the non-volatile memory 103 and that maintains the target site concentration of the drug after delivery at the target concentration stored in the non-volatile memory 103. The target site concentration of the drug after delivery can be calculated by simulation on the basis of the amount of delivered drug, patient information and the type of drug to be delivered, stored in the non-volatile memory 103. Delivery of the drug is performed by pressing of the syringe plunger 202, similarly to step S103.

Next, step S204 calculates the target site concentration of the delivered drug by simulation on the basis of the amount of drug delivered in step S203, patient information stored in the non-volatile memory 103, or the like.

Step S205 judges whether the target site concentration calculated in step S204 has reached the target concentration stored in the non-volatile memory 103. In a case where it is determined that the target concentration has not been reached, processing returns to step S202. In a case where it is determined that the target concentration has been reached, processing moves on to step S206.

Step S208 performs liquid delivery with a delivery flow rate that does not exceed the upper limit value stored in the non-volatile memory 103 and that maintains the target site concentration of the drug after delivery, at the target concentration stored in the non-volatile memory 103. The target site concentration of the drug after delivery can be calculated by simulation similarly to step S204. Delivery of the drug is performed by pressing of the syringe plunger 202, similarly to step S103.

Next, step S209 calculates the target site concentration of the delivered drug by simulation on the basis of the amount of drug delivered in step S208, patient information stored in the non-volatile memory 103, or the like.

Step S210 determines whether the target site concentration calculated in step S209 has reached the target concentration stored in the non-volatile memory 103. In a case where it is determined that the target concentration has been reached, processing returns to step S207. In a case where it is determined that the target concentration has not been reached, processing moves on to step S211.

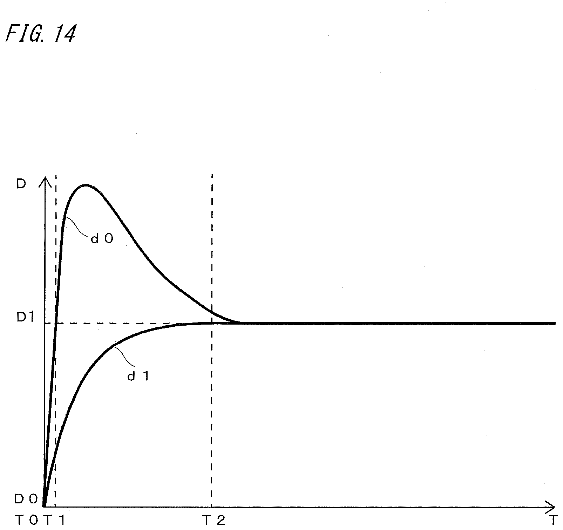

Fig. 14 is a graph illustrating an exemplary change in the concentration of a drug in the living body when the drug is delivered using the syringe pump according to the second embodiment. In this, d0 represents a drug concentration in blood, d1 represents an target site concentration, and D1 represents a target concentration. T0 represents delivery start time, T1 represents the time at which the drug concentration in blood reaches the target concentration D1, and T2 represents the time at which the target site concentration reaches the target concentration D1.

At time T0, the syringe pump according to the second embodiment starts delivery of the drug while setting the upper limit of the initial flow rate as the upper limit value of the delivery flow rate. After starting liquid delivery, the drug concentration in blood d0 reaches the target concentration D1 at time T1. When the liquid delivery is continued, the target site concentration d1 reaches the target concentration D1. At this time, the upper limit value of the delivery flow rate is switched from the upper limit of the initial flow rate to the upper limit of the maintenance flow rate. On and after time T2, the target site concentration is maintained at the target concentration.

Unlike the case of the first embodiment, the drug concentration in blood d0 is not maintained at the target concentration during a period from time T1 to time T2. The reason is that, while the delivery flow rate is adjusted such that the drug concentration in blood is maintained at a target concentration in the first embodiment, the delivery flow rate is adjusted such that the target site concentration d1 is maintained at the target concentration in the second embodiment. As a result, the delivery flow rate during the period from time T1 to time T2 is greater in the second embodiment relative to the first embodiment. Accordingly, it is possible to reduce the time needed for the target site concentration to reach the target concentration, compared with the first embodiment.

According to the present embodiment, the delivery flow rate at the time of delivery of the drug is adjusted so as to maintain the target site concentration at a target concentration. This makes it possible to promptly allow the target site concentration to reach the changed target concentration, and thus, to allow the efficacy of the drug to be expressed more promptly.

In the above-described second embodiment, the control unit switches the upper limit value when the simulated target site concentration reaches the target concentration. However, the configuration is not limited to this. For example, the control unit of the syringe pump according to the second embodiment may be configured such that the upper limit value is switched when the simulated drug concentration in blood reaches the target concentration. With this configuration of the control unit of the syringe pump according to the second embodiment, it is possible to deliver the drug while adjusting the delivery flow rate more appropriately according to an applied technique and the type of drug.

### <Third Embodiment>

Next, a syringe pump according to a third embodiment of the present invention will be described.

In the syringe pump according to the third embodiment, operation related to switching of the upper limit value of the delivery flow rate and the operation related to adjustment of delivery flow rate, performed by the control unit, differ from the operation of the control unit 100 of the syringe pump according to the first embodiment and its modification, and from the operation of the control unit of the syringe pump according to the second embodiment. In the control unit 100 of the syringe pump according to the first embodiment and its modification and in the control unit of the syringe pump according to the second embodiment, the concentration as a reference of switching the upper limit value of the delivery flow rate and the concentration to be maintained at the target concentration are prescribed. Meanwhile, the control unit of the syringe pump according to the third embodiment differs from the control unit 100 of the syringe pump according to the first embodiment and its modification and from the control unit of the syringe pump according to the second embodiment, in that it is possible to specify the concentration as a reference for switching the upper limit value of the delivery flow rate and the concentration to be maintained at a target concentration.

For units other than the control unit, namely, for the main body cover 2, the display unit 3, the operation panel unit 4, or the like, the configuration is the same as the configuration in the first embodiment, and thus, description will be omitted. Among the operation of the control unit of the syringe pump according to the third embodiment, operation including drive of the motor driver 134 and reception of input of target concentration is the same as the operation in the first embodiment, and thus, description will be omitted.

Hereinafter, switching of the upper limit value of the delivery flow rate and operation related to adjustment of the delivery flow rate, performed by the control unit of the syringe pump according to the third embodiment, will be described. Note that operation of determining whether liquid delivery conditions are satisfied, operation of delivering with the delivery flow rate below the upper limit value, or the like, are same as the operation of the control unit 100 of the syringe pump according to the first embodiment and its modification and from the control unit of the syringe pump according to the second embodiment, and thus, description will be omitted.

The control unit of the syringe pump according to the third embodiment switches the upper limit of the delivery flow rate according to whether the concentration specified as a reference for switching the upper limit value of the delivery flow rate has reached the target concentration.

Specifically, in a case where the target site concentration is specified as a concentration as a reference for switching the upper limit value of the delivery flow rate, the control unit of the syringe pump according to the third embodiment switches the upper limit of the delivery flow rate according to whether the simulated target site concentration has reached the target concentration. In another case where the drug concentration in blood is specified as a concentration as a reference for switching the upper limit value of the delivery flow rate, the control unit of the syringe pump according to the third embodiment switches the upper limit of the delivery flow rate according to whether the simulated drug concentration in blood has reached the target concentration.

In addition, the control unit of the syringe pump according to the third embodiment adjusts the delivery flow rate so as to maintain the concentration specified as the concentration to be maintained at a target concentration, at the target concentration.

Specifically, in a case where the target site concentration is specified as a concentration to be maintained at a target concentration, the control unit of the syringe pump according to the third embodiment adjusts the delivery flow rate so as to maintain the simulated target site concentration at the target concentration. In another case where the drug concentration in blood is specified as a concentration to be maintained at a target concentration, the control unit of the syringe pump according to the third embodiment adjusts the delivery flow rate so as to maintain the simulated drug concentration in blood at the target concentration.

It is possible to specify, onto the control unit 100, concentration as a reference concentration for switching the upper limit value of the delivery flow rate, and the concentration to be maintained at a target concentration, with various methods.

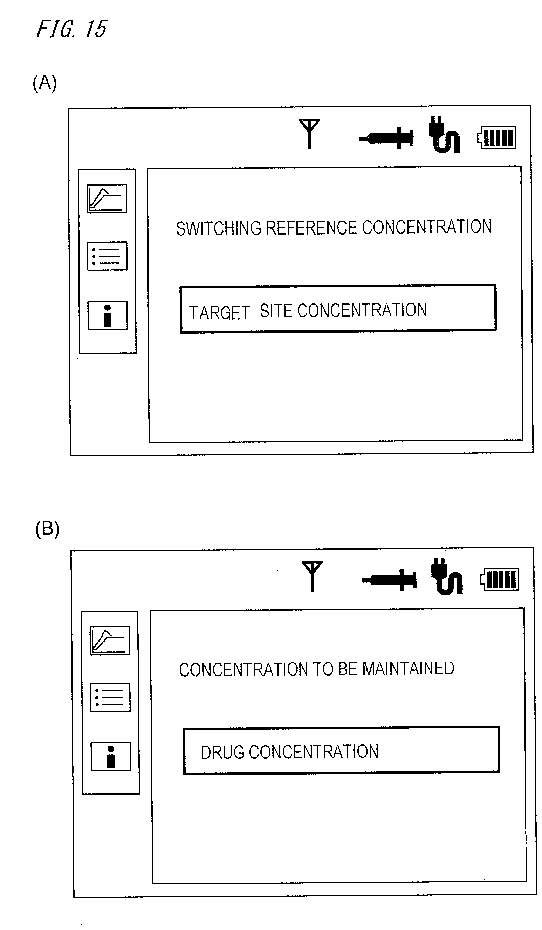

For example, by operating operation buttons on the operation panel unit 4 according to the exemplary displayed information on the display unit 3 illustrated in Figs. 15(A) and (B), it is possible to specify, onto the control unit 100, the concentration as a reference for switching the upper limit value of the delivery flow rate, and the concentration to be maintained at a target concentration.

According to the present embodiment, it is possible to facilitate appropriate setting of the timing for switching the upper limit value of the delivery flow rate and the concentration to be maintained at a target concentration, according to an applied technique and the types of drugs, and thus, to further enhance safety and convenience.

While the syringe pump according to the present invention is described with embodiments and modifications, the syringe pump according to the present invention is not limited to these configurations, but can by variously modified on the basis of the description of the claims.

While the above-described embodiments and modifications describe a case where the present invention is applied to a syringe pump, application is not limited to this exemplary case. The present invention can be broadly applied to medical liquid delivery pumps including an infusion pump capable of adjusting the delivery flow rate of a drug.

This application is on the basis of Japanese Patent Application No. 2014-058596, filed on March 20, 201 4.

### Reference Signs List

- 1: syringe pump
- 2: main body cover
- 2A: upper portion of main body cover
- 2B: lower portion of main body cover
- 3: display unit
- 4: operation panel unit
- 5: clamp
- 6: syringe setting unit
- 7: syringe plunger drive unit
- 8: container
- 9: tube fixing unit
- 100: control unit
- 103: non-volatile memory
- 200, 300, 400: syringe
- 201, 301, 401: syringe main body
- 202, 302, 402: syringe plunger

## Claims

1. A liquid delivery pump configured to deliver a drug while simulating a concentration of the delivered drug in a living body, the liquid delivery pump comprising:
a calculation unit configured to calculate a simulated concentration of the drug in blood and a simulated target site concentration in the living body on the basis of the amount of the drug delivered by the liquid delivery pump;
a determination unit configured to provide a determination result whether one of the simulated drug concentration of the drug in blood and a simulated target site concentration of the drug, calculated by the calculation unit, has reached a predetermined target concentration; wherein
a storage unit configured to store an upper limit of an initial flow rate of the drug of the liquid delivery pump determined for individual types of drugs, an upper limit of a maintenance flow rate of the drug of the liquid delivery pump determined for individual types of drugs, and the target concentration;
a reception unit configured to receive input of the upper limit of the initial flow rate, the upper limit of the maintenance flow rate, and the target concentrations;
an upper limit value switching unit configured to switch an upper limit value of a delivery flow rate of the drug to one of the upper limit of the initial flow rate and the upper limit of the maintenance flow rate, according to the determination result from the determination unit,
wherein in a case where the upper limit of the initial flow rate is set as the upper limit value, the upper limit value switching unit switches the upper limit value to the upper limit of the maintenance flow rate when the determination unit has determined that one of the simulated drug concentration of the drug in the blood and the simulated target site concentration has reached the target concentration,
wherein in a case where the upper limit of the maintenance flow rate is set as the upper limit value, the upper limit value switching unit switches the upper limit value to the upper limit of the initial flow rate when the determination unit has determined that one of the simulated drug concentration of the drug in the blood and the simulated target site concentration has not reached the target concentration; and
an adjustment unit configured to adjust the delivery flow rate of the drug in a range that does not exceed the upper limit value.

2. The liquid delivery pump according to claim 1,
wherein the adjustment unit adjusts the delivery flow rate of the drug such that the delivery flow rate does not exceed the upper limit value and such that the simulated drug concentration of the drug in blood is maintained at the target concentration.

3. The liquid delivery pump according to any one of claims 1 to 2,
wherein the adjustment unit adjusts the delivery flow rate of the drug such that the delivery flow rate does not exceed the upper limit value and such that the target site concentration is maintained at the target concentration.

4. The liquid delivery pump according to any one of claims 1 to 3,
wherein the reception unit further receives input of the upper limit of the initial flow rate and the upper limit of the maintenance flow rate, and limits reception of input of the upper limit of the maintenance flow rate to not exceed the upper limit of the initial flow rate.

5. The liquid delivery pump according to any one of claims 1 to 4,
wherein the reception unit receives input of the target concentration during liquid delivery.

6. The liquid delivery pump according to any one of claims 1 to 5,
wherein the determination unit determines that one of the simulated drug concentration in blood and the target site concentration has reached the simulated target concentration when one of the simulated drug concentration in blood and the simulated target site concentration is included in a predetermined tolerance range on a bases of the target concentration.

7. The liquid delivery pump according to any one of claims 1 to 6, comprising a reporting unit configured to report to a user that the upper limit value has been switched.

8. A software for controlling operation of a liquid delivery pump (1) for delivering a drug while simulating a concentration of the delivered drug in a living body, comprising executable instructions for processing by a control unit (100) of said liquid delivery pump (1), said instructions including:
- setting (S 101, S201)) an upper limit of an initial flow rate as an upper limit value of a delivery flow rate according to a type of drug to be delivered,
- determining (S102, S202)) whether a liquid delivery finish condition is satisfied, and if the liquid delivery finish condition is satisfied, finishing the liquid delivery, otherwise
- performing (S103, S203) liquid delivery with a delivery flow rate that does not exceed the upper limit value for maintaining the drug concentration of the drug in blood or a target site concentration after delivery at the target concentration, and calculating the drug concentration of the drug in blood or the target site concentration after delivery by simulation on the basis of the amount of delivered drug, patient information and the type of drug to be delivered
calculating (S104, S204) the target site concentration of the delivered drug by simulation on the basis of the amount of drug delivered (S103), patient information and the type of drug to be delivered,
determining (S105, S205) whether the target site concentration calculated (S104, S204) has reached the target concentration, and if it is determined that the target concentration has not been reached, processing returns to determining (S102, S202) . whether a liquid delivery finish condition is satisfied, otherwise
switching (S106, S206) the upper limit value of the delivery flow rate from the upper limit of the initial flow rate to the upper limit of the maintenance flow rate, and optionally reporting that the upper limit value has been switched,
determining (S107, S207) whether a liquid delivery finish condition is satisfied, and if the liquid delivery finish condition is satisfied, finishing the liquid delivery, otherwise
performing (S108, S208) liquid delivery with the delivery flow rate that does not exceed the upper limit value for maintaining the drug concentration in blood or the target site concentration, after delivery, of the drug at the target concentration, wherein the drug concentration in blood, or the target site concentration, after delivery, of the drug is calculated by simulation,
calculating (S109, S209) the target site concentration of the delivered drug by simulation on the basis of the amount of drug delivered (S 108, S208), patient information and the type of drug to be delivered,
determining (S110, S210) whether the target site concentration calculated (S109, S209) has reached the target concentration, and if it is determined that the target concentration has been reached, processing returns to determining (S107, S207), otherwise
switching (S111, S211) the upper limit value of the delivery flow rate from the upper limit of the maintenance flow rate to the upper limit of the initial flow rate, and optionally reporting that the upper limit value has been switched, and returning to determining (S102, S202) whether a liquid delivery finish condition is satisfied; and
- repeating the afore processing until the liquid delivery finish condition is satisfied.

## Patentansprüche

1. Flüssigkeitsabgabepumpe, die so ausgelegt ist, dass sie ein Medikament verabreicht und dabei eine Konzentration des verabreichten Medikaments in einem lebenden Körper simuliert, wobei die Flüssigkeitsabgabepumpe Folgendes umfasst:
eine Berechnungseinheit, die so ausgelegt ist, dass sie eine simulierte Konzentration des Medikaments im Blut und eine simulierte Konzentration an der Zielstelle im lebenden Körper auf der Grundlage der Menge des von der Flüssigkeitsabgabepumpe abgegebenen Medikaments berechnet;
eine Ermittlungseinheit, die so ausgelegt ist, dass sie ein Ermittlungsergebnis darüber liefert, ob die simulierte Wirkstoffkonzentration des Medikaments im Blut oder eine simulierte Konzentration des Medikaments an der Zielstelle, die von der Berechnungseinheit berechnet wurde, eine vorbestimmte Zielkonzentration erreicht hat; wobei
eine Speichereinheit, die so ausgelegt ist, dass sie eine Obergrenze einer Anfangsflussrate des Medikaments der Flüssigkeitsabgabepumpe, die für einzelne Arten von Medikamenten bestimmt wurde, eine Obergrenze einer Aufrechterhaltungsflussrate des Medikaments der Flüssigkeitsabgabepumpe, die für einzelne Arten von Medikamenten bestimmt wurde, und die Zielkonzentration speichert;
eine Aufnahmeeinheit, die so ausgelegt ist, dass sie die obere Grenze der anfänglichen Durchflussrate, die obere Grenze der Aufrechterhaltungsflussrate und die Zielkonzentrationen erfasst;
eine Umschalteinheit für den oberen Grenzwert, die so ausgelegt ist, dass sie einen oberen Grenzwert einer Abgabeflussrate des Medikaments auf den oberen Grenzwert der anfänglichen Durchflussrate oder den oberen Grenzwert der Aufrechterhaltungsflussrate umschaltet, entsprechend dem Ermittlungsergebnis der Ermittlungseinheit,
in einem Fall, in dem der obere Grenzwert der anfänglichen Durchflussrate als oberer Grenzwert festgelegt ist, die Umschalteinheit für den oberen Grenzwert diesen auf den oberen Grenzwert der Aufrechterhaltungsflussrate umschaltet, wenn die Ermittlungseinheit festgestellt hat, dass entweder die simulierte Wirkstoffkonzentration des Medikaments im Blut oder die simulierte Konzentration an der Zielstelle die Zielkonzentration erreicht hat,
in einem Fall, in dem der obere Grenzwert der Aufrechterhaltungsflussrate als oberer Grenzwert festgelegt ist, die Umschalteinheit für den oberen Grenzwert diesen auf den oberen Grenzwert der anfänglichen Flussrate umschaltet, wenn die Ermittlungseinheit festgestellt hat, dass entweder die simulierte Wirkstoffkonzentration des Medikaments im Blut oder die simulierte Konzentration an der Zielstelle die Zielkonzentration nicht erreicht hat; und
eine Verstelleinheit, die so ausgelegt ist, dass sie die Abgabeflussrate des Medikaments in einem Bereich einstellt, der den oberen Grenzwert nicht überschreitet

2. Flüssigkeitsabgabepumpe nach Anspruch 1,
wobei die Verstelleinheit die Abgabeflussrate des Medikaments so einstellt, dass die sie den oberen Grenzwert nicht überschreitet und dass die simulierte Wirkstoffkonzentration des Medikaments im Blut auf der Zielkonzentration gehalten wird.

3. Flüssigkeitsabgabepumpe nach einem der Ansprüche 1 bis 2,
wobei die Verstelleinheit die Abgabeflussrate des Medikaments so einstellt, dass die sie den oberen Grenzwert nicht überschreitet und dass die Konzentration an der Zielstelle auf der Zielkonzentration gehalten wird.

4. Flüssigkeitsabgabepumpe nach einem der Ansprüche 1 bis 3,
wobei die Aufnahmeeinheit ferner die Eingabe der Obergrenze der anfänglichen Durchflussrate und der Obergrenze der Aufrechterhaltungsflussrate aufnimmt und die Aufnahme der Eingabe der Obergrenze der Aufrechterhaltungsflussrate so begrenzt, dass sie die Obergrenze der anfänglichen Durchflussrate nicht überschreitet.

5. Flüssigkeitsabgabepumpe nach einem der Ansprüche 1 bis 4,
wobei die Aufnahmeeinheit während der Flüssigkeitsabgabe eine Eingabe der Zielkonzentration erhält.

6. Flüssigkeitsabgabepumpe nach einem der Ansprüche 1 bis 5,
wobei die Ermittlungseinheit feststellt, dass entweder die simulierte Wirkstoffkonzentration im Blut oder die Konzentration an der Zielstelle die simulierte Zielkonzentration erreicht hat, wenn entweder die simulierte Wirkstoffkonzentration im Blut oder die simulierte Konzentration an der Zielstelle in einem vorbestimmten Toleranzbereich auf der Grundlage der Zielkonzentration enthalten ist.

7. Flüssigkeitsabgabepumpe nach einem der Ansprüche 1 bis 6, mit einer Meldeeinheit, die so ausgelegt ist, dass sie einem Benutzer meldet, dass der obere Grenzwert umgeschaltet wurde.

8. Eine Software zur Funktionssteuerung einer Flüssigkeitsabgabepumpe für die Verabreichung eines Medikaments (1) während der Simulation einer Konzentration des abgegebenen Medikaments in einem lebenden Körper, die ausführbare Anweisungen zur Verarbeitung durch eine Steuereinheit (100) der Flüssigkeitsabgabepumpe (1) aufweist, wobei diese Anweisungen Folgendes beinhalten:
- Einstellen (S 101, 5201) einer Obergrenze einer anfänglichen Durchflussrate als oberer Grenzwert einer Abgabeflussrate entsprechend einer zu verabreichenden Art von Medikament,
- Bestimmen (5102, 5202), ob eine Endbedingung für die Flüssigkeitsabgabe erfüllt ist, und sofern diese erfüllt ist, Beenden der Flüssigkeitsabgabe, andernfalls
- Durchführen (5103, 5203) einer Flüssigkeitsabgabe mit einer Abgabeflussrate, die den oberen Grenzwert nicht überschreitet, um die Wirkstoffkonzentration des Medikaments im Blut oder eine Konzentration an der Zielstelle nach der Abgabe auf der Zielkonzentration zu halten, und die Berechnung der Wirkstoffkonzentration des Medikaments im Blut oder der Konzentration an der Zielstelle nach der Verabreichung durch Simulation auf der Grundlage der Menge des verabreichten Medikaments, der Patienteninformationen und der Art des zu verabreichenden Medikaments
Berechnung (5104, 5204) der Konzentration des verabreichten Medikaments an der Zielstelle durch Simulation auf der Grundlage der Menge des verabreichten Medikaments (5103), der Patienteninformationen und der Art des zu verabreichenden Medikaments,
Bestimmen (5105, 5205), ob die berechnete (5104, 5204) Konzentration an der Zielstelle die Zielkonzentration erreicht hat, und falls festgestellt wird, dass diese nicht erreicht wurde, geht die Verarbeitung zur Bestimmung (5102, 5202) zurück, egal ob eine Bedingung für die Flüssigkeitsabgabe erfüllt ist, andernfalls
Umschalten (5106, 5206) des oberen Grenzwerts der Durchflussmenge von der oberen Grenze der anfänglichen Durchflussmenge auf die obere Grenze der Aufrechterhaltungsflussmenge, und gegebenenfalls Meldung, dass der obere Grenzwert umgeschaltet wurde,
Bestimmen (5107, 5207), ob eine Endbedingung für die Flüssigkeitsabgabe erfüllt ist, und sofern diese erfüllt ist, Beenden der Flüssigkeitsabgabe, andernfalls
Durchführen (5108, 5208) einer Flüssigkeitsabgabe mit der Abgabeflussrate, die den oberen Grenzwert nicht überschreitet, um die Konzentration des Medikaments im Blut oder die Konzentration des Medikaments an der Zielstelle nach der Abgabe auf der Zielkonzentration zu halten, wobei die Konzentration des Medikaments im Blut oder die Konzentration des Medikaments an der Zielstelle nach der Abgabe durch Simulation berechnet wird,
Berechnung (5109, 5209) der Konzentration des verabreichten Medikaments an der Zielstelle durch Simulation auf der Grundlage der Menge des verabreichten Medikaments (S 108, 5208), der Patienteninformationen und der Art des zu verabreichenden Medikaments,
Bestimmen (5110, 5210), ob die berechnete (5109, 5209) Konzentration an der Zielstelle die Zielkonzentration erreicht hat, und falls festgestellt wird, dass diese erreicht wurde, geht die Verarbeitung zur Bestimmung (5107, 5207) zurück, andernfalls
Umschalten (5111, 5211) des oberen Grenzwerts der Abgabeflussrate von der oberen Grenze der Aufrechterhaltungsflussrate auf die obere Grenze der anfänglichen Durchflussrate und gegebenenfalls Melden, dass der obere Grenzwert umgeschaltet wurde, sowie Zurückgehen zur Bestimmung (5102, 5202), ob eine Bedingung zur Flüssigkeitsabgabe erfüllt ist; und
- Wiederholung des vorgenannten Verfahrens, bis die Bedingung für die Flüssigkeitsabgabe erfüllt ist.

## Revendications

1. Pompe d'administration de liquide configurée pour administrer un médicament tout en simulant une concentration du médicament administré dans un corps vivant, la pompe d'administration de liquide comprenant :
une unité de calcul configurée pour calculer une concentration simulée du médicament dans le sang et une concentration sur site cible simulée dans le corps vivant sur la base de la quantité du médicament administré par la pompe d'administration de liquide ;
une unité de détermination configurée pour fournir un résultat de détermination concernant le fait qu'une concentration parmi la concentration de médicament simulée du médicament dans le sang et une concentration sur site cible simulée du médicament, calculées par l'unité de calcul, a atteint ou non une concentration cible prédéterminée ; où
une unité de mémorisation configurée pour mémoriser une limite supérieure d'un débit initial du médicament de la pompe d'administration de liquide déterminé pour des types individuels de médicaments, une limite supérieure de débit d'entretien du médicament de la pompe d'administration de liquide déterminé pour des types individuels de médicaments, et la concentration cible ;
une unité de réception configurée pour recevoir une entrée de la limite supérieure du débit initial, de la limite supérieure du débit d'entretien, et des concentrations cibles ;
une unité de changement de valeur de limite supérieure configurée pour changer une valeur de limite supérieure d'un débit d'administration du médicament en une limite parmi la limite supérieure du débit initial et la limite supérieure du débit d'entretien, selon le résultat de détermination de l'unité de détermination,
où dans un cas où la limite supérieure du débit initial est fixée comme étant la valeur de limite supérieure, l'unité de changement de valeur de limite supérieure change la valeur de limite supérieure en la limite supérieure du débit d'entretien lorsque l'unité de détermination a déterminé qu'une concentration parmi la concentration de médicament simulée du médicament dans le sang et la concentration sur site cible simulée a atteint la concentration cible,
où dans un cas où la limite supérieure du débit d'entretien est fixée comme étant la valeur de limite supérieure, l'unité de changement de valeur de limite supérieure change la valeur de limite supérieure en la limite supérieure du débit initial lorsque l'unité de détermination a déterminé qu'une concentration parmi la concentration de médicament simulée du médicament dans le sang et la concentration sur site cible simulée n'a pas atteint la concentration cible ; et
une unité de réglage configurée pour régler le débit d'administration du médicament dans une plage qui ne dépasse pas la valeur de limite supérieure.

2. Pompe d'administration de liquide selon la revendication 1,
dans laquelle l'unité de réglage règle le débit d'administration du médicament de telle sorte que le débit d'administration ne dépasse pas la valeur de limite supérieure et de telle sorte que la concentration de médicament simulée du médicament dans le sang est maintenue à la concentration cible.

3. Pompe d'administration de liquide selon l'une quelconque des revendications 1 et 2,
dans laquelle l'unité de réglage règle le débit d'administration du médicament de telle sorte que le débit d'administration ne dépasse pas la valeur de limite supérieure et de telle sorte que la concentration sur site cible est maintenue à la concentration cible.

4. Pompe d'administration de liquide selon l'une quelconque des revendications 1 à 3,
dans laquelle l'unité de réception reçoit en outre une entrée de la limite supérieure du débit initial et de la limite supérieure du débit d'entretien, et limite une réception d'entrée de la limite supérieure du débit d'entretien de sorte à ne pas dépasser la limite supérieure du débit initial.

5. Pompe d'administration de liquide selon l'une quelconque des revendications 1 à 4,
dans laquelle l'unité de réception reçoit une entrée de la concentration cible durant l'administration de liquide.

6. Pompe d'administration de liquide selon l'une quelconque des revendications 1 à 5,
dans laquelle l'unité de détermination détermine qu'une concentration parmi la concentration de médicament simulée dans le sang et la concentration sur site cible a atteint la concentration cible simulée lorsqu'une concentration parmi la concentration de médicament simulée dans le sang et la concentration sur site cible simulée est incluse dans une plage de tolérance prédéterminée sur la base de la concentration cible.

7. Pompe d'administration de liquide selon l'une quelconque des revendications 1 à 6, comprenant une unité de rapport configurée pour rapporter à un utilisateur que la valeur de limite supérieure a été changée.

8. Logiciel destiné à commander un fonctionnement d'une pompe d'administration de liquide (1) afin d'administrer un médicament tout en simulant une concentration du médicament administré dans un corps vivant, comprenant des instructions exécutables destinées à être traitées par une unité de commande (100) de ladite pompe d'administration de liquide (1), lesdites instructions incluant :
- fixer (S101, 5201) une limite supérieure d'un débit initial en tant que valeur de limite supérieure d'un débit d'administration selon un type de médicament à administrer,
- déterminer (S102, S202) si une condition de fin d'administration de liquide est satisfaite ou non, et si la condition de fin d'administration de liquide est satisfaite, mettre un terme à l'administration de liquide, sinon
- effectuer (S103, S203) une administration de liquide avec un débit d'administration qui ne dépasse pas la valeur de limite supérieure pour maintenir la concentration de médicament du médicament dans le sang ou une concentration sur site cible après administration à la concentration cible, et calculer la concentration de médicament du médicament dans le sang ou la concentration sur site cible après administration par simulation sur la base de la quantité de médicament administré, d'informations sur le patient et du type de médicament à administrer
calculer (S104, S204) la concentration sur site cible du médicament administré par simulation sur la base de la quantité de médicament administré (S103), d'informations sur le patient et du type de médicament à administrer,
déterminer (S105, S205) si la concentration sur site cible calculée (S104, S204) a atteint ou non la concentration cible, et s'il est déterminé que la concentration cible n'a pas été atteinte, retourner à l'étape consistant à déterminer (S102, S202) si une condition de fin d'administration de liquide est satisfaite ou non, sinon
changer (S 106, S206) la valeur de limite supérieure du débit d'administration de la limite supérieure du débit initial à la limite supérieure du débit d'entretien, et optionnellement rapporter que la valeur de limite supérieure a été changée,
déterminer (S107, S207) si une condition de fin d'administration de liquide est satisfaite ou non, et si la condition de fin d'administration de liquide est satisfaite, mettre un terme à l'administration de liquide, sinon
effectuer (S108, S208) une administration de liquide avec le débit d'administration qui ne dépasse pas la valeur de limite supérieure pour maintenir la concentration de médicament dans le sang ou la concentration sur site cible, après administration, du médicament à la concentration cible, où la concentration de médicament dans le sang, ou la concentration sur site cible, après administration, du médicament est calculée par simulation,
calculer (S109, S209) la concentration sur site cible du médicament administré par simulation sur la base de la quantité de médicament administré (S108, S208), d'informations sur le patient et du type de médicament à administrer,
déterminer (S110, S210) si la concentration sur site cible calculée (S109, S209) a atteint ou non la concentration cible, et s'il est déterminé que la concentration cible a été atteinte, retourner à l'étape de détermination (S107, S207) sinon
changer (S111, S211) la valeur de limite supérieure du débit d'administration de la limite supérieure du débit d'entretien à la limite supérieure du débit initial, et optionnellement rapporter que la valeur de limite supérieure a été changée, et retourner à l'étape consistant à déterminer (S102, S202) si une condition de fin d'administration de liquide est satisfaite ou non ; et
- recommencer le traitement susmentionné jusqu'à ce que la condition de fin d'administration de liquide soit satisfaite.
